(19) Europäisches Patentamt
European Patent Office
Office européen des brevets

(11) Numéro de publication: **0 330 591**
**A1**

(12) # DEMANDE DE BREVET EUROPEEN

(21) Numéro de dépôt: **89420064.1**

(22) Date de dépôt: **21.02.89**

(51) Int. Cl.$^4$: **C 07 C 125/065**
C 07 C 125/073

(30) Priorité: **23.02.88 FR 8802440**

(43) Date de publication de la demande:
**30.08.89 Bulletin 89/35**

(84) Etats contractants désignés:
**BE DE ES FR GB IT**

(71) Demandeur: **RHONE-POULENC CHIMIE**
**25, quai Paul Doumer**
**F-92408 Courbevoie Cédex (FR)**

(72) Inventeur: **Leconte, Philippe**
**30, Rue Moenchsberg**
**F-68100 Mulhouse (FR)**

**Metz, François**
**22, Rue de Condé**
**F-69002 Lyon (FR)**

(74) Mandataire: **Varnière-Grange, Monique et al**
**RHONE-POULENC INTERSERVICES Service Brevets**
**Chimie Centre de Recherches de Saint-Fons B.P. 62**
**F-69192 Saint-Fons Cédex (FR)**

(54) **Procédé de préparation de carbamates aromatiques.**

(57) L'invention concerne un perfectionnement à un procédé de préparation de carbamates aromatiques par réaction d'au moins un composé nitroaromatique, du monoxyde de carbone et d'au moins un composé organique porteur d'au moins un groupe hydroxyle en présence

a) d'un catalyseur à base de palladium,

b) d'au moins un coordinat de formule :

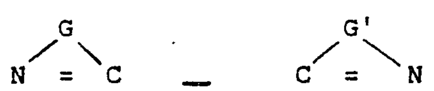

(I)

dans laquelle G et G′, identiques ou différents, représentent chacun un groupe pontant qui comporte 3 ou 4 atomes dont au moins 2 sont des atomes de carbone, G et G′ pouvant par ailleurs être reliés l'un à l'autre, et,

c) d'au moins un acide protonique dont aucun proton n'est relié à un atome d'halogène. Le perfectionnement comprend le fait que la quantité de palladium présente dans le milieu réactionnel est inférieure ou égale à 0,01 mole par litre dudit milieu et qu'on opère en respectant au moins l'une des conditions suivantes :

- le rapport molaire ($H^+$/Pd) est supérieur à 150,
- le rapport molaire (coordinat/Pd) est supérieur à 20.

Le procédé selon l'invention permet notamment de fabriquer des toluylènecarbamates de dialkyle, précurseurs des diisocyanatotoluènes.

EP 0 330 591 A1

## Description

### PROCEDE DE PREPARATION DE CARBAMATES AROMATIQUES

La présente invention a pour objet un procédé de préparation de carbamates aromatiques par réaction d'au moins un composé nitroaromatique, du monoxyde de carbone et d'au moins un composé organique porteur d'au moins un groupe hydroxyle en présence d'un catalyseur à base de palladium.

Les carbamates en cause (ou uréthanes) sont des précurseurs d'isocyanates, les isocyanates étant d'un emploi très courant, par exemple dans la production de polyuréthanes. Parmi les isocyanates les plus importants on citera tout particulièrement le diisocyanatotoluène (TDI) et le méthylènediphényldiisocyanate (MDI) qui sont jusqu'à ce jour produits industriellement par phosgénation des diamines correspondantes, et se présentent souvent sous forme de mélanges d'isomères.

Il a déjà été proposé divers procédés de réduction de composés nitroaromatiques par du monoxyde de carbone et un alcanol inférieur en présence de divers systèmes catalytiques qui présentent tous l'un au moins des inconvénients majeurs ci-après :
- présence d'impuretés métalliques indésirables dans le produit réactionnel.
- risque élevé de corrosion de l'appareillage par des ions chlorures provenant le plus souvent des chlorures ferreux ou ferriques engagés dans la réaction comme co-catalyseurs.

Plus récemment il a également été proposé un procédé de préparation de carbamates aromatiques par réaction d'au moins un composé nitroaromatique, du monoxyde de carbone et d'au moins un composé organique porteur d'au moins un groupe hydroxyle en présence d'un catalyseur à base de palladium. En effet dans la demande de brevet européen EP-A-0086281 il est décrit un tel procédé dont la caractéristique proposée comprend le recours à des classes particulières de coordinats dont des représentants typiques sont le tétraphényldiphosphinoéthane, la bipyridyle-2,2′ et la phénanthroline-1,10 et qui, pratiquement sont engagés à raison de 0,02 à 20 moles par atome-gramme de palladium présent dans le milieu réactionnel. Dans cette même demande il est également proposé de conduire la réaction en cause en présence d'un acide en vue d'augmenter la vitesse de la réaction et, le cas échéant la sélectivité en carbamate recherché ; ces acides sont pratiquement engagés à raison de 0,01 à 150 équivalents par atome-gramme de palladium présent dans le milieu réactionnel.

Toutefois, si l'intérêt de principe d'une telle technique n'est nullement contesté, son développement à l'échelle industrielle est compromis par les quantités relativement importantes de palladium ou de composés du palladium qu'il s'avère nécessaire d'engager à la réaction pour obtenir une activité convenable du système catalytique et une sélectivité acceptable en produit.

Nonobstant le coût du métal catalytique, qui rend nécessaire le recyclage du catalyseur, et le caractère versatile dudit métal ou de ces composés, le recours à des quantités importantes d'un tel métal ou de ces composés rend le recyclage de celui-ci ou de ceux-ci difficile et augmente le risque de voir le métal se déposer notamment sur les parois de l'appareillage. Il apparaît donc souhaitable de pouvoir conduire la réaction en cause avec des quantités limitées de palladium ou de composés du palladium. Or, lorsqu'on limite les quantités en cause pour atteindre au maximum une concentration exprimée en mole de palladium par litre de milieu réactionnel de l'ordre de 0,01 mole par litre, on observe, dans le cadre de la technique décrite dans la demande européenne en cause, à la fois une baisse rédhibitoire de la sélectivité en produit recherché et une chute notable de l'activité.

Il était donc nécessaire de proposer une solution permettant de diminuer la quantité de palladium ou de composés du palladium engagée dans une telle réaction sans pour autant observer les inconvénients précités.

Il a maintenant été trouvé en procédé de préparation de carbamates aromatiques par réaction d'au moins un composé nitroaromatique, du monoxyde de carbone et d'au moins un composé organique porteur d'au moins un groupe hydroxyle en présence d'un catalyseur à base de palladium, d'au moins un coordinat de formule

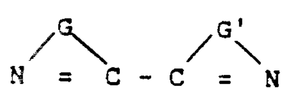

(I)

dans laquelle G et G′, identiques ou différents, représentent chacun un groupe pontant qui comporte 3 ou 4 atomes dont au moins 2 sont des atomes de carbone, G et G′ pouvant par ailleurs être reliés l'un à l'autre, et d'au moins un acide protonique dont aucun proton n'est relié à un atome d'halogène, caractérisé en ce que la quantité de palladium présente dans le milieu réactionnel est inférieure ou égale à 0,01 mole par litre dudit milieu et en ce qu'on opère en respectant au moins l'une des conditions suivantes :
- le rapport molaire ($H^+$/Pd) est supérieur à 150
- le rapport molaire (coordinat/Pd) est supérieur à 20

Le procédé selon la présente invention comprend la mise en réaction d'au moins un composé nitroaromatique c'est-à-dire un composé comportant au moins un groupe $NO_2$ relié directement à un atome de carbone, cet atome de carbone étant un maillon d'un cycle aromatique - Parmi les composés

nitroaromatiques utilisables comme matière première dans le procédé en cause on peut citer le nitrobenzène et ses homologues obtenus par substitution d'un ou plusieurs atomes d'hydrogène par un/ou des groupes alkyles, alcoxy, aryles ou aryloxy ; les dinitrobenzènes et leur homologues obtenus par substitution d'un ou plusieurs atomes d'hydrogène par un ou des groupes alkyles, alcoxy, aryles ou aryloxy et les polynitrobenzènes. On citera plus particulièrement les nitrotoluènes, les dinitrotoluènes, le dinitro-4,4′ diphényl méthane. Bien entendu des mélanges de deux ou plusieurs composés nitroaromatiques peuvent être utilisés et en particulier les mélanges des isomères -2,4 et -2,6 du dinitrotoluène.

Au moins un composé nitroaromatique est mis à réagir avec du monoxyde de carbon et au moins un composé organique porteur d'au moins un groupe hydroxyle, ces composés hydroxylés pouvant être représentés par la formule générale (II) ci-après,

$R^1(OH)_n$ (II)

dans laquelle :

- n est un entier non nul et au maximum égal à 4
- $R^1$ représente un radical alkyle, ou arakyle, qui peut être substitué par un ou plusieurs atomes d'halogène et/ou groupes alcoxy comportant au maxium 4 atomes de carbone, et qui renferme au maximum 20 atomes de carbone, et, de préférence au maximum 6 atomes de carbon.

A titre d'exemples de composés monohydroxylés répondant à la formule générale (II) on peut citer : le méthanol, l'éthanol, le n-propanol et le propanol secondaire, l'hexanol-1, le dodécanol-1, l'hexadécanol-1, l'alcool chlorobenzylique, l'alcool méthoxybenzylique, le butoxyéthanol, le cyclohexanol et le trifluoroéthanol.

A titre d'exemples de composés polyhydroxylés répondant à la formule générale (II) ci-avant on peut citer : l'éthylèneglycol, le diéthylèneglycol, le propylèneglycol, le glycérol et le triméthylolpropane.

Bien entendu des mélanges de deux ou plusieurs composés peuvent être utilisés.

Conviennent plus particulièrement bien à la mise en oeuvre du présent procédé le méthanol, l'éthanol, le propanol, l'isopropanol et l'éthylène glycol ; les alcanols comportant au maximum 4 atomes de carbone sont avantageusement utilisés dans le cadre de la présente invention.

Le rapport entre les quantités du composé nitroaromatique et du composé organique hydroxylé n'est pas critique : l'un comme l'autre peuvent être utilisé en excès. En pratique il s'avère commode d'opérer avec un excès de composé organique hydroxylé qui sert alors de solvant de réaction. Il est également possible de conduire la réaction dans un diluant inerte tel un hydrocarbure aliphatique ou aromatique éventuellement halogéné, et, notamment le benzène, le toluène, le chlorobenzène ou l'hexane.

Le procédé selon l'invention requiert la présence de palladium ou de composés du palladium.

N'importe quelle source de palladium peut être utilisée dans la mise en oeuvre dudit procédé. En effet, on peut recourir à du palladum métallique, pris tel quel ou déposé sur un support inerte tel le noir de carbone ou l'alumine, à des sels ou complexes du palladium.

Dans les conditions de la réaction en cause la majorité de ces sources de palladium sera soluble dans le milieu réactionnel. A titre d'exemples de composés du palladium convenant à la mise en oeuvre du présent procédé on peut citer :

- les carboxylates de palladium dont l'anion comporte, de préférence au maximum 12 atomes de carbone et en particulier l'acétate de palladium et le propionate de palladium ;
- les halogénures de palladium et en particulier le chlorure, de palladium et le bromure de palladium ;
- l'acétylacétonate de palladium, les complexes du palladium et de la dibenzylidèneacétone tel $Pd(dba)_3$.

L'acétate de palladium convient particulièrement bien à la mise en oeuvre du procédé selon l'invention.

Comme indiqué en tête du présent mémoire la concentration en palladium dans le milieu réactionnel est inférieure à 0,01 mole par litre. Cette concentration est avantageusement inférieure à 0,0025 mole par litre. Cette concentration est en général au moins égale à $10^{-4}$ mole/litre pour obtenir une transformation appréciable du (ou des) composé (s) nitroaromatique(s).

Le procédé selon la présente invention nécessite la présence dans le milieu réactionnel d'au moins un coordinat de formule (I), rappelée ci-après dans laquelle G et G′ ont la signification indiquée précédemment :

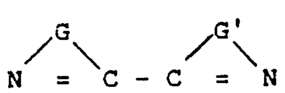

Lorsque le(s) groupe(s) pontant(s) G et/ou G′ comporte(nt) un(ou deux) atome(s) distinct(s) des atomes de carbone, ces atomes sont de préférence des atomes d'azote. G et G′ peuvent en outre être reliés entre eux par un groupe de deux atomes de carbone pour former un coordinat de formule (I) présentant le squelette de la phénanthroline-1,10. A titre d'exemples de coordinats de formule générale (I) on peut citer : le bipyridyle-2,2′ le diméthyl-4,4′ bipyridyle-2,2′, le diméthoxy-4,4′ bipyridyle-2,2′, le dicarboxy-4,4′ bipyridyle-2,2′, le dichloro-4,4′ bipyridyle-2,2′, le biquinolyle-2,2′, la phénanthroline-1,10, la diphényl-4,7 phénanthroline-1,10, la tétraméthyl-3,4,7,8 phénanthroline-1,10, et la diméthyl-4,7 phénanthroline-1,10.

La phénanthroline-1,10 et ses dérivés non substitués sur les positions 2 et/ou 9 conviennent plus

particulièrement comme coordinats dans le cadre du présent procédé.

On recourt, de préférence, à la phénanthroline-1,10.

La quantité de coordinat à mettre en oeuvre peut varier dans de larges limites lorsque par ailleurs la quantité d'acide protonique engagée est telle que le rapport molaire $H^+/Pd$ est supérieur à 150. En général la quantité de coordinat est d'au moins une mole par mole de palladium. Pour une bonne mise en oeuvre de la présente invention elle est supérieure à 20 moles par mole de palladium. Aucun avantage n'est observé lorsque le rapport molaire (coordinat/palladium) excède 2000. De préférence,ce rapport molaire compris entre 100 et 1500.

Le procédé selon l'invention requiert en outre la présence d'un acide protonique dont aucune proton n'est relié à un atome d'halogène. De nombreuses catégories d'acides protoniques conviennent à la mise en oeuvre dudit procédé et à titre d'exemples on peut citer :

- $HBF_4$, $HPF_6$, $H_2SiF_6$ et plus généralement les acides formés, le cas échéant in situ, par réaction d'HF sur un acide de Lewis tels $BF_3$ et $PF_5$,
- $HClO_4$
- les acides sulfoniques et en particulier l'acide triméthyl-2,4,6 benzène sulfonique
- les acides carboxyliques tel l'acide acétique, l'acide mésitoïque ou l'acide trifluoroacétique.

De préférence, on recourt à un acide protonique de pka supérieur à 2, non estérifiable ou difficilement estérifiable dans les conditions de la réaction. L'acide mésitoïque convient particulièrement bien à la mise en oeuvre du procédé.

La quantité d'acide protonique à mettre en oeuvre dans le cadre du présent procédé peut varier dans de très larges limites lorsque par ailleurs la quantité engagée de coordinat au sens précédemment défini est telle que le rapport molaire (coordinat/Pd) est supérieur à 20. En général la quantité d'acide protonique est d'au moins 0,01 mole par mole de palladium. Aucun avantage n'est observé lorsque ledit rapport molaire excède 5000. Pour une bonne mise en oeuvre de la présente invention, le rapport molaire $H^+/Pd$ est supérieur à 150. De préférence, ledit rapport molaire est compris entre 200 et 2500.

Comme indiqué en tête du présent mémoire, il est essentiel dans le cadre du présent procédé que l'une au moins des deux conditions ci-après, visant des rapports molaires, soit respectée :

- $H^+/Pd > 150$
- Coordinat/Pd $> 20$

De préférence, ces deux conditions sont simultanément remplies. De bons résultats sont par ailleurs observés lorsque l'un et/ou l'autre de ces rapports molaires se trouve supérieur aux valeurs respectives préférées, déjà indiquées ci-avant, et rappelées pour plus de commodité comme suit :

- $H^+/Pd > 200$
- Coordinat/Pd $> 100$,

Bien entendu aucun avantage n'est observé à dépasser les valeurs respectives suivantes :

- pour $H^+/Pd$ : 5000 et, de préférence : 2500
- pour coordinat/Pd : 2000 et, de préférence : 1500.

Le procédé est généralement conduit à une température d'au moins 90°C pour observer un résultat appréciable et, de préférence, cette température reste inférieure à 200°C pour limiter les risques de dégradatation des produits formés et des matières premières.

Dans la majorité des cas on obtient une activité raisonnable et des résultats satisfaisants lorsque la température de réaction se situe dans l'intervalle de 120 à 200°C. De très bons résultats peuvent être obtenus dans la plage de température de 130 à 180°C. Le procédé est normalement conduit sous une pression supérieure à la pression atmosphérique, la pression pouvant atteindre 500 bar (50000 KPa).

De préférence, la pression est comprise entre 10 et 150 bar (1000 et 15000 KPa). De bons résultats peuvent être obtenus avec une pression comprise entre 30 et 120 bar (3000 et 12000 KPa).

A la fin de l'opération ou après un temps de séjour souhaité les produits sont récupérés par tout moyen approprié. Par exemple, le mélange réactionnel peut être soumis à une distillation et/ou à une cristallisation.

Les exemples ci-après illustrent l'invention ; les conventions suivantes y sont utilisées :

- Pd mmol/1 : désigne la concentration en palladium.
- L/Pd : désigne le rapport molaire du coordinat au palladium.
- TT(%) : désigne le taux de transformation du composé nitroaromatique.
- RT(%) : désigne le nombre de moles de carbamate formé pour 100 moles de composé nitroaromatique converties.

Exemple 1 - Essais témoins (a) et (b) :

Dans un autoclave de 300 ml en acier inoxydable on charge 130 ml d'une solution renfermant sauf mention contraire :

- du nitrobenzène (1,2 mol/l)
- de la phénanthroline-1,10 (0,03 mol/l)
- de l'acide mésitoïque (0,08 mol/l)
- de l'acétate de palladium
- de l'éthanol (q.s.p. 1 l)

On balaye alors l'air de l'autoclave par du monoxyde de carbone puis on admet une pression de 120 bar (12 000 KPa) de CO. On porte alors la température à 180°C sous agitation tout en maintenant la pression à sa

valeur précédemment indiquée. Après une durée en température désignée par t ci-après, le mélange réactionnel est refoidi, dégazé puis analysé par chromatographie en phase gazeuse et en phase liquide.

Les conditions particulières ainsi que les résultats obtenus figurent dans le tableau I ci-après :

TABLEAU I :

| Réf. | Pd mmol/1 | L/Pd | H+/Pd | t mn | T T (0/0) | R T (0/0) |
|---|---|---|---|---|---|---|
| a | 7.6 | 4 | 10.5 | 45 | 100 | 77 |
| b | 2 | 15 | 40 | 90 | 100 | 68 |
| 1(*) | 0,50 | 1000 | 2000 | 30 | 100 | 70 |

(*) dans cet essai les concentrations en phénantholine-1,10 et en acide mésitoïque sont respectivement les suivantes : 0,5 mol/1 et 1 mol/1.

L'essai témoin (a) est représentatif de l'enseignement de l'art antérieur lorsque le procédé est mis en oeuvre avec une concentration relativement forte en palladium.

L'essai témoin (b) demontre qu'avec une concentration moindre en palladium et dans les conditions de l'art antérieur on observe à la fois une baisse de l'efficacité (il faut plus de temps pour obtenir le même taux de conversion) et une baisse de la sélectivité en produit recherché.

L'exemple (1) réalisé avec une faible concentration en palladium mais en mettant conjointement en oeuvre des rapport molaires L/Pd et $H^+$/Pd élevés, entrant dans le cadre de la présente invention, met en évidence l'augmentation de l'activité et la sauvegarde d'une sélectivité appréciable.

Exemples 2 à 5 ; essais témoins (c) et (d) :

Dans un autoclave en tantale de 125 ml de capacité on charge 20 ml d'une solution renfermant :
- du nitrobenzène (1,2 mol/l)
- de la phénanthroline-1,10
- de l'acide trifluoroacétique
- de l'acétate de palladium
- du méthanol (q.s.p. 1 l)

On utilise un mode opératoire analogue à celui décrit pour l' exemple 1 ci-avant, la température étant de 140°C, la pression de 60 bars (6000 KPa) et la durée en température de chaque essai de 4 heures. Les conditions particulières ainsi que les résultats obtenus figurent dans le tableau II ci-après :

TABLEAU II :

| Réf. | Pd mmol/1 | L/Pd | H+/Pd | T T (0/0) | R T (0/0) |
|---|---|---|---|---|---|
| c | 5 | 20 | 150 | 100 | 21 |
| d | 0,5 | 20 | 150 | 58 | 40 |
| 2 | 0,5 | 20 | 750 | 67 | 16 |
| 3 | 0,5 | 200 | 150 | 96 | 68 |
| 4 | 0,5 | 600 | 150 | 100 | 79 |
| 5 | 0,5 | 200 | 750 | 100 | 44 |
| e | 2,5 | 20 | 150 | 100 | 54 |
| 6(*) | 2,5 | 120 | 150 | 100 | 61 |

(*) t = 90 mn

Exemple 6 ; Essai témoin (e) :

Ces deux essais dont les résultats figurent également au tableau II ont été réalisés dans des conditions similaires à celles décrites pour les exemples 2 à 5 à ceci près que la température est de 180° C dans les deux essais et que la durée de l'exemple 6 n'a pas dépassé 90 minutes la conversion étant totale dès ce moment là.

Exemples 7 à 10 ; Essai témoin (f) :

Dans un autoclave en tantale de 125 ml de capacité on charge 20 ml d'une solution renfermant :
- du nitrobenzène (1,2 mol/l)
- de la phénanthroline-1,10
- de l'acide mésitoïque
- de l'acétate de palladium (0,5 mmol/l)
- du méthanol (q.s.p. 1 l)

On utilise un mode opératoire analogue à celui décrit pour les exemples 1 et 2 ci-avant, la température étant

de 140°C, la pression de 60 bar (6000 KPa) et la durée en température de 4 h.
Les conditions particulières et les résultats obtenus figurent dans le tableau III ci-après :

TABLEAU III

| Réf. | L/Pd | H+/Pd | T T (%) | R T (%) |
|---|---|---|---|---|
| f | 20 | 150 | 24 | 55 |
| 7 | 100 | 150 | 48 | 67 |
| 8 | 600 | 150 | 100 | 75 |
| 9 | 20 | 300 | 21 | 67 |
| 10 | 20 | 1200 | 31 | 39 |

Exemple 11 à 13 ; essais témoins (g) et (h) :

Dans l'autoclave en tantale et selon un mode opératoire analogue à celui utilisé précédemment on réalise une série d'essais au départ de 20 ml d'une solution renfermant :

- du nitrobenzène (1,2 mol/l)
- de la phénanthroline-1,10
- de l'acide mésitoïque
- du palladium sous forme du complexe Pd (Phén.) $(OAc)_2$ (Phén : phénanthroline-1,10 (1,9 mmol/l)
- de l'éthanol (q.s.p. 1 l)

La température est de 180°C et la pression de 120 bar (12 000 KPa).
Les conditions particulières ainsi que les résultats obtenus figurent dans le tableau IV ci-après :

TABLEAU IV :

| Réf. | L/Pd | H+/Pd | t mn | T T (%) | R T (%) |
|---|---|---|---|---|---|
| g | 11,5 | 20 | 65 | 99 | 41 |
| 11 | 11,5 | 202,3 | 60 | 96 | 82 |
| h | 15,8 | 40,5 | 75 | 100 | 70 |
| 12 | 15,8 | 202,5 | 60 | 100 | 82 |
| 13 | 15,8 | 405 | 40 | 100 | 85 |

Exemple 14:

On reproduit l'exemple 9 ci-avant en ne modifiant que la nature de la source de palladium qui est maintenant le complexe $Pd(dba)_3$ (dba : dibenzylydène acétone) et le rapport molaire de la phénanthroline-1,10 au palladium qui est maintenant de 30

TT = 27 %
RT = 64 %

Exemple 15 :

Dans l'autoclave en tantale et selon un mode opératoire analogue à celui décrit précédemment on réalise un essai sur une charge de 20 ml constituée par :

- 24 mmol de nitrobenzène
- 0,01 mAtg de palladium sous forme d'acétate de palladium
- 10 mmol de phénanthroline-1,10
- 2 mmol d'acide trifluoroacétique
- (q.s.p. 20 ml) de méthanol

Les conditions sont les suivantes :

Pd = 0,5 mmol/l
L/Pd = 1000 ; $H^+$/Pd = 200
Température = 180°C
Pression = 120 bar (12000 KPa)

Les résultats obtenus après deux heures en température sont les suivants :

TT = 100 %
RT = 62 %

Exemple 16 :

Dans l'autoclave en tantale et selon un mode opératoire analogue à celui décrit précédemment on réalise un essai une charge de 20 ml constituée par :

- 24 mmol de nitrobenzène
- 0,04 mAtg de palladium sous forme d'acétate de palladium
- 1 mmol de bipyridyle-2,2′
- 7,5 mmol d'acide mésitoïque
- (q.s.p. 20 ml) de méthanol

Les conditions sont les suivantes :

Pd = 2,0 mmol/l

L/Pd = 25 ; $H^+$/Pd = 187

Température = 140°C

Pression = 60 bar (6 000 KPa)

Les résultats obtenus après deux heures en température sont les suivantes :

TT = 47 %

RT = 70 %

Eexemple 17 :

Dans l'autoclave en tantale et selon un mode opératoire analogue à celui décrit précédemment on réalise un essai sur une charge de 20 ml constituée par :

- 12 mmol de dinitro-2,4 toluène
- 0,04 mAtg de palladium sous forme d'acétate de palladium
- 1 mmol de phénanthroline-1,10
- 7,5 mmol d'acide mésitoïque
- de méthanol (q.s.p. 20 ml)

Les conditions sont les suivantes :

Pd = 2.0 mmol/l

L/Pd = 25 ; $H^+$/Pd = 187

Température = 140°C

Pression = 60 bar (6 000 KPa)

Les résultats obtenus après 4 h de réaction en température sont les suivants :

. Transformation du dinitrotoluène : 100 %

. Sélectivité en toluylènecarbamate de diméthyle : 60 %

**Revendications**

1° - Procédé de préparation de carbamates aromatiques par réaction d'au moins un composé nitroaromatique, du monoxyde de carbone et d'au moins un composé organique porteur d'au moins un group hydroxyle en présence

a) d'un catalyseur à base de palladium,

b) d'au moins un coordinat de formule :

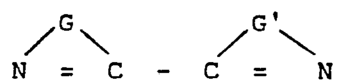

(I)

dans laquelle G et G′, identiques ou différents, représentent chacun un groupe pontant qui comporte 3 ou 4 atomes dont au moins 2 sont des atomes de carbone, G et G′ pouvant par ailleurs être reliés l'un à l'autre, et,

c) d'au moins un acide protonique dont aucun proton n'est relié à un atome d'halogène, caractérisé en ce que la quantité de palladium présente dans le milieu réactionnel est inférieure ou égale à 0,01 mole par litre dudit milieu et en ce qu'on opère en respectant au moins l'une des conditions suivantes :

- le rapport molaire ($H^+$/Pd) est supérieur à 150,
- le rapport molaire (coordinat/Pd) est supérieur à 20

2° - Procédé selon la revendication 1 caractérisé en ce qu'on opère avec un rapport molaire ($H^+$/Pd) supérieur à 150 et avec un rapport molaire (coordinat/Pd) supérieur à 20.

3° - Procédé selon la revendication 1 ou 2 caractérisé en ce que le rapport molaire $H^+$/Pd est inférieur à 5000.

4° - Procédé selon l'une quelconque des revendications précédentes, caractérisé en ce que le rapport molaire (coordinat/Pd) est inférieur à 2000.

5° - Procédé selon l'une quelconque des revendications précédentes, caractérisé en ce que le rapport molaire $H^+$/Pd est compris entre 200 et 2500.

6° - Procédé selon l'une quelconque des revendications précédentes, caractérisé en ce que le rapport molaire (coordinat/Pd) est compris entre 100 et 1500.

7° - Procédé selon l'une quelconque des revendications précédentes, caractérisé en ce que la concentration au palladium dans le milieu réactionnel est inférieure à 0,0025 mole par litre.

8° - Procédé selon l'une quelconque des revendications précédentes, caractérisé en ce que le coordinat utilisé est la phénanthroline-1,10.

9° - Procédé selon l'une quelconque des revendications précédentes, caractérisé en ce que l'acide protonique utilisé est l'acide mésitoïque.

10° - Procédé selon l'une quelconque des revendications précédentes, caractérisé en ce que la température de réaction est comprise entre 120 et 200° C et, de préférence, entre 130 et 180° C.

11° - Procédé selon l'une quelconque des revendications précédentes, caractérisé en ce que la pression de réaction est comprise entre 10 et 150 bar (1000 et 15 000 KPa) et, de préférence entre 30 et 120 bar (3000 et 12000 KPa).

12° - Procédé selon l'une quelconque des revendications précédentes, caractérisé en ce qu'on utilise comme composé organique porteur d'au moins un groupe hydroxyle un alcanol comportant au maximum 4 atomes de carbone.

13° - Procédé selon l'une quelconque des revendications précédentes, caractérisé en ce qu'on utilise comme composé nitroaromatique un composé choisi parmi le nitrobenzène, les dinitrotoluènes ou leurs mélanges.

Office européen des brevets

# RAPPORT DE RECHERCHE EUROPEENNE

Numero de la demande

EP 89 42 0064

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
| --- | --- | --- | --- |
| D,X | EP-A-0 086 281 (SHELL INTERNATIONALE RESEARCH MAATSCHAPPIJ)<br>* En entier * | 1-13 | C 07 C 125/065<br>C 07 C 125/073 |
| X | EP-A-0 169 650 (MONTEDISON)<br>* En entier * | 1-13 | |
| X | EP-A-0 231 045 (SHELL INTERNATIONALE RESEARCH MAATSCHAPPIJ)<br>* En entier * | 1-13 | |

DOMAINES TECHNIQUES RECHERCHES (Int. Cl.4)

C 07 C 125/00

Le présent rapport a été établi pour toutes les revendications

| Lieu de la recherche | Date d'achèvement de la recherche | Examinateur |
| --- | --- | --- |
| LA HAYE | 31-05-1989 | WELLS A.G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant